# EUROPÄISCHE PATENTSCHRIFT

(11) **EP 1 056 705 B1**
(45) Veröffentlichungstag und Bekanntmachung des Hinweises auf die Patenterteilung: **29.10.2003**
(21) Anmeldenummer: 99908845.3
(22) Anmeldetag: 04.02.1999
(51) Int. Cl.: C07C 50/36, A61K 35/78

(54) **STABILE HYPERFORIN-SALZE, VERFAHREN ZU IHRER HERSTELLUNG UND VERWENDUNG ZUR THERAPIE DER ALZHEIMERSCHEN KRANKHEIT**
STABLE HYPERFORIN SALTS, METHOD FOR PRODUCING SAME AND THEIR USE IN THE TREATMENT OF ALZHEIMER'S DISEASE
SELS D'HYPERFORINE STABLES, LEUR PROCEDE DE PREPARATION ET LEUR UTILISATION POUR LE TRAITEMENT DE LA MALADIE D'ALZHEIMER

(30) Priorität: 13.02.1998 DE 19805947
(43) Veröffentlichungstag der Anmeldung: 06.12.2000
(73) Patentinhaber: Dr. Willmar Schwabe GmbH & Co. KG, 76227 Karlsruhe (DE)
(72) Erfinder: CHATTERJEE, Shyam, Sunder, D-76139 Karlsruhe (DE); ERDELMEIER, Clemens, D-76139 Karlsruhe (DE); KLESSING, Klaus, D-76275 Ettlingen (DE); MARME, Dieter, D-79104 Freiburg (DE); SCHÄCHTELE, Christoph, D-79108 Freiburg (DE)
(74) Vertreter: Bunke, Holger, Dr.rer.nat. Dipl.-Chem.
(86) Internationale Anmeldenummer: EP9900737
(87) Internationale Veröffentlichungsnummer: WO99041220

(56) Entgegenhaltungen:
- EP-A- 0 599 307
- EP-B- 1 054 682
- WO-A-97/13489

## Beschreibung

### Gegenstand der Erfindung :

Gegenstand der Erfindung ist die Bereitstellung stabiler Salze des Hyperforins und Adhyperforins, welche als solche oder durch Freisetzung des Hyperforins bzw. Adhyperforins pharmakologisch wirksam werden können. Ein wesentlicher Aspekt der Erfindung betrifft die Bereitstellung eines Verfahrens zur Anreicherung bzw. Reingewinnung des Hyperforins und Adhyperforins aus Johanniskraut-Extrakten mittels Ausfällung in Form solcher stabiler Salze. Ein weiterer besonders bedeutsamer Gegenstand der Erfindung besteht darin, neue Wirkstoffe gegen die Alzheimersche Krankheit (im folgenden abgekürzt als "AD"), die einen kausaltherapeutischen Ansatz aufweisen, bereitzustellen.

Ein weiterer wichtiger Aspekt der vorliegenden Erfindung betrifft die Bereitstellung von Wirkstoff-Kombinationen, die nicht nur zur kausalen Therapie der AD angewandt werden können, sondern gleichzeitig auch die bei der AD häufig auftretenden psychopathologischen Begleiterscheinungen wie Ängstlichkeit, Depressionen und cognitive Störungen beseitigen, erheblich bessern oder zumindest deren Progression aufhalten können.

### Hintergrund der Erfindung:

Das amyloide Peptid Aβ1-42, ein Prozessierungsprodukt des Alzheimer Precursor Proteins APP, spielt eine zentrale Rolle bei der Entstehung der AD [Lamb, B.T.: Presenilins, amyloid-β and Alzheimer's disease. Nature Med. 3 (1997) 28-29. Selkoe, D.J.: Alzheimer's Disease: Genotypes, Phenotype, and Treatments. Science 275 (1997) 630-631.]. Diese Hypothese wird gestützt durch folgende experimentelle Befunde:

APP Missense-Mutationen (Patienten mit familiärer AD) führen zu erhöhter Freisetzung von Aβ1-42 [Scheuner, D. et al.: Secreted amyloid β-protein similar to that in the senile plaques of Alzheimer's disease is increased in vivo by the presenilin 1 and 2 and APP mutations linked to familial Alzheimer's disease. Nature Med. 2 (1996) 864-870.].

Mutationen in Presenilin 1 und Presenilin 2 (Patienten mit familiärer AD) führen ebenfalls zu einem Anstieg an freigesetztem Aβ1-42 [Scheuner, D. et al]. Transgene Mäuse, die mutiertes APP überexprimieren, entwickeln altersabhängige Ablagerungen von Aβ und zeigen kognitive Störungen [Games, D. et al.: Alzheimer-type neuropathology in transgenic mice overexpressing V717F β-amyloid precursor protein. Nature 373 (1995) 523-527. Hsiao, K. et al.: Correlative Memory Deficits, Aβ Elevation, and Amyloid Plaques in Transgenic Mice. Science 274 (1996) 99-102.].

Die proteolytische Spaltung des pathogenen Aβ aus dem Alzheimer Precursor Protein APP wird vermittelt durch die β- und γ-Sekretase, deren molekulare Identität nicht bekannt ist. Die α-Sekretase prozessiert APP zu einer löslichen Form (sAPP) und einem cytoplasmatischen Rest. Der Schnitt der α-Sekretase liegt innerhalb von Aβ, so daß in diesem Fall kein pathogenes Aβ entsteht. Die molekulare Identität der α-Sekretase ist ebenfalls nicht bekannt.

Die α-Sekretase wird stimuliert durch Acetylcholin, vermittelt durch die muscarinischen Rezeptoren m1 und m3 [Nitsch, R.M. et al.: Release of Alzheimer amyloid precursor derivatives stimulated by activation of muscarinic acetyl-choline receptors. Science 258 (1992) 304-307]. Zellulärer Mediator ist die Proteinkinase C ("PKC"). Dies belegen auch Experimente, die nach direkter Stimulierung der PKC durch Phorbolester zum gleichen Ergebnis kommen [Buxbaum, J.D. et al.: Processing of Alzheimer beta/A4 amyloid precursor protein: Modulation by agents that regulate protein phosphorylation. Proc. Natl. Acad. Sci. USA 87 (1990) 6003-6006].

Tacrin, das bislang erfolgreichste Therapeutikum gegen die AD ist ein Acetylcholinesterase-Inhibitor [Giacobini, E.: Cholinomimetic therapy of Alzheimer disease: Does it slow down deterioration? In Recent Advances in the Treatment of Neurodegenerative Disorders and Cognitive Dysfunction, Int. Acad. Biomed. Drug Res. 7 (1994) 51-57. Racagni, G. et al., eds. Basel: Karger].

Das läßt sich interpretieren als eine indirekte Stimulierung der α-Sekretase durch folgende Signalkette: Tacrin hemmt die Acetylcholinesterase. Dadurch wird die Konzentration von Acetylcholin erhöht. Acetylcholin aktiviert über die muscarinischen Rezeptoren m1 und m3 die PKC. Dadurch wird die Aktivität der α-Sekretase erhöht. Als Folge wird die Menge an pathogenem Aβ erniedrigt.

Aus diesen Befunden lässt sich ableiten, daß selektive Aktivierung der PKC ein wirksamer therapeutischer Ansatz zur Hemmung der Produktion von amyloidogenem Aβ und damit zur Behandlung der AD sein kann. Da von allen 11 PKC-Isoenzymen die γ-Form als einziger Subtyp ausschließlich in neuronalen Zellen exprimiert wird, stellen Substanzen, die die PKC-γ stimulieren, einen neuen Ansatzpunkt zur Therapie der AD dar. Darüber hinaus sind alle Substanzen oder Prozesse, die die α-Sekretase stimulieren bzw. die β- und γ-Sekretase inhibieren, zur Verhinderung der Freisetzung von pathogenem Aβ und damit zur kausalen Therapie der AD geeignet.

### Stand der Technik:

Das Phloroglucin-Derivat Hyperforin ist einer der Hauptinhaltsstoffe in frischem Johanniskraut. Es wird in geringerer Konzentration begleitet von seinem Homologen Adhyperforin. Da beide Substanzen sehr instabil gegenüber Licht- und Lufteinfluss sind, nimmt ihr Gehalt bereits bei der Trocknung der Frischpflanze ab. Durch schnelle und schonende Trocknung, gefolgt von geeigneten Extraktionsverfahren können Extrakte mit Gehalten von etwa 3-60% Hyperforin/Adhyperforin gewonnen werden [DE 19619512 C1]. 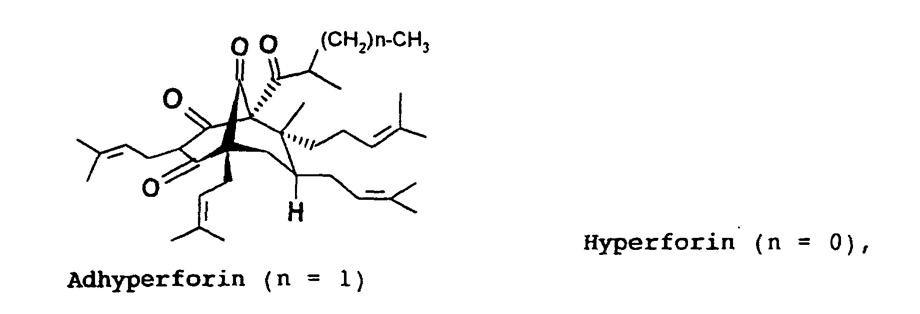 Ohne Zusatz geeigneter Stabilisierungsmittel ist das Hyperforin jedoch nicht stabil und kann daher nur unter hohem technischen Aufwand in angereicherter oder reiner Form gewonnen und gelagert werden.

In EP-A-0599307 wurde bereits auf die Bedeutung des Hyperforins zur Erzielung der antidepressiven Wirksamkeit von Johanniskraut-Extrakten hingewiesen. Inzwischen wurde wissenschaftlich belegt, dass Hyperforin aufgrund seines pharmakologischen Profils einen wesentlichen Einfluss bei der medizinischen Behandlung von Depressionen und weiterer Serotononin-abhängiger Erkrankungen ausübt [S.S. Chatterjee et al., Hyperforin and Hypericum extract, Interactions with some Neurotransmitter Systems (SL-82), 2nd Intern. Congress on Phytomedicine, Sept. 11-14, 1996, München. Siehe auch: Pharmacopsychiatry 1998, 31 Suppl.I, 1-60].

Die Alzheimer-Demenz (AD) ist eine schwerwiegende, schleichend beginnende Krankheit, die besonders in fortgeschrittenem Alter einen erheblichen Anteil der Bevölkerung befällt. Sie ist gekennzeichnet durch anfängliche Vergesslichkeit, dann zunehmende Gedächtnisstörungen und Einbußen bei weiteren kognitiven Fähigkeiten. Sie endet mit dem völligen geistigen Verfall und Persönlichkeitsverlust und verläuft letzlich lethal. Es steht bislang keine befriedigende, kausal orientierte Therapie der AD zur Verfügung [K. Mendla, Die Alzheimer-Krankheit: Neue Ansätze in der Pharmakotherapie (1996). Pharm.Ztg. 141, 351-356].

### Technisches Problem.

Das der Erfindung zugrunde liegende technische Problem besteht demnach darin, dass zum einen keine technisch befriedigende Methode zur Gewinnung und Stabilisierung von reinem bzw. stark angereichertem Hyperforin und Adhyperforin bekannt ist, wodurch die Isolierung, Lagerung und Verwendung dieser Substanzen sehr behindert ist, und dass zum anderen ein Mangel an Wirkstoffen zur kausal orientierten Therapie der Alzheimerschen Krankeit besteht, wodurch immense Kosten im menschlichen Sozialbereich entstehen. Der Erfindung liegt die Aufgabe zugrunde, diesen Mängeln abzuhelfen.

### Lösung des technischen Problems.

Diese Aufgabe wird erfindungsgemäß gelöst durch
- die neuen Salze des Hyperforins und Adhyperforins gemäß den Patentansprüchen 1 bis 5;
- das Verfahren zur Herstellung dieser Salze gemäß Patentanspruch 6;
- das Verfahren zur Anreicherung bzw. Reingewinnung von Hyperforin und Adhyperforin in Form dieser Salze, gemäß den Patentansprüchen 7 und 8;
- die Verwendung dieser Salze zur stabilen Lagerhaltung von Hyperforin, Adhyperforin und deren Gemischen, gemäß Patentanspruch 9;
- die pharmazeutische Zubereitung gemäß Patentanspruch 10 sowie
- die neue Verwendung von Adhyperforin und Gemischen aus Hyperforin and Adhyperforin als Arzneimittel zur Behandlung der AD (2. medizinische Indikation).

Es wurde überraschenderweise gefunden, dass die Instabilität des Hyperforins bzw. des Adhyperforins durch Überführung desselben in geeignete Salze der allgemeinen Formel I

[A⁻]ₘ [B]^{p+} (I)

vollständig aufgehoben oder zumindest erheblich vermindert werden kann. Salze des Hyperforins sind bisher nicht bekannt.

Hierbei bedeuten in Formel I m eine ganze Zahl von 1 bis 3 und

[A⁻]

das Anion des Hyperforins bzw. des Adhyperforins, wobei n = 0 oder 1 ist (allgemeine Formel II): 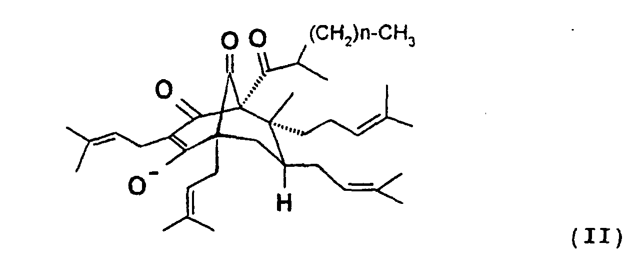 und [B]^{p+} bedeutet entweder
ein Alkalimetallion, vorzugsweise Li⁺, Na⁺ oder K⁺, wobei p = 1 ist,
oder ein Ammoniumion einer salzbildenden Stickstoffbase der allgemeinen Formel **III**, 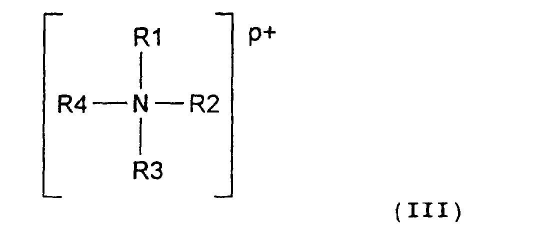 worin die Reste **R1, R2 und R3**,
unabhängig voneinander, ein Wasserstoffatom, eine geradkettige oder verzweigte Alkyl-, Cycloalkyl-, Bicycloalkyl-, Tricycloalkyl-, Alkenyl-, Alkinyl-, Heterocyclalkyl-, Aryl-, Heteroaryl, Arylalkyl- oder Heteroarylalkyl-Gruppe oder ein durch einen oder mehrere Hydroxy-, Alkoxy-, Aryloxy-, Alkanoyl-, Aroyl-, Carboxy-, Alkoxycarbonyl-, Amino-, Alkylamino-, Hydroxylamino-, Amido-, Carbamoyl-, Ureido-, Amidino-, Guanidino-, Cyano-, Azido-, Mercapto-, Alkylthio-, Alkylsulfoxy-Alkylsulfonyl-, Alkylsulfenyl-, Aminosulfonyl-, Fluor-, Chlor-, Brom-, Jod-, Alkyl- oder Perfluoralkyl-Rest(e) substituiertes Derivat der genannten Gruppen bedeuten,
oder worin die Reste **R1 und R2**
zusammen mit dem N-Atom einen Azetidin-, Pyrrolidin-, Pyrrolin-, Piperidin-, Piperazin-, Homopiperazin-, Morpholin-, Thiomorpholin-, Pyridin-, Di- oder Tetrahydropyridin-, Pyrimidin-, Pyrazin-, Azepin-, Dihydroazepin-, Oxazepin-, Diazepin-, Imidazol-, Pyrazol-, Oxazol- oder Thiazol-Ring oder einen der genannten Ringe bedeuten, der ankondensierte aliphatische, heteroaliphatische, aromatische oder hetero-aromatische Ringe aufweist und/oder durch einen oder mehrere Hydroxy-, Alkoxy-, Aryloxy-, Alkanoyl-, Aroyl-, Carboxy-, Alkoxycarbonyl-, Amino-, Alkylamino-, Hydroxylamino-, Amido-, Carbamoyl-, Ureido-, Amidino-, Guanidino-, Cyano-, Azido-, Mercapto-, Alkylthio-, Alkylsulfoxy-, Alkylsulfonyl-, Alkylsulfenyl-, Aminosulfonyl-, Fluor-, Chlor-, Brom-, Jod-, Alkyl- oder Perfluoralkyl-Rest(e) substituiert ist,
und worin der Rest **R4**
ein Wasserstoffatom oder eine geradkettige oder verzweigte Alkylgruppe bedeutet,
wobei **p** = **m** ist und die Gesamtanzahl positiver Ladungen des Rests [B] angibt.

Besonders bevorzugt sind die N,N-Dicyclohexylamin-Salze des Hyperforins und Adhyperforins sowie deren Gemische.

Aus der oben angeführten Definition der als Salzbildner dienenden Base B geht hervor, dass eine Vielzahl basischer Stickstoffverbindungen dazu geeignet ist, die Stabilität des in ungeladener Form instabilen Hyperforins bzw. Adhyperforins in befriedigender Weise zu erhöhen.

Geeignete Basen sind z. B.:
- ggf. durch eine oder mehrere Hydroxylgruppen substituierte aliphatische und cycloaliphatische Amine oder Polyamine wie mono-, di- oder tri-(C₃ bis C₂₀)-Alkylamine, Aminoethanol, Methylaminoethanol, Dimethylaminoethanol, Cholin, 2-Hydroxy-1.1-dimethylethylamin, Tris-(hydroxymethyl)-methylamin, N-Methyl-D-glucamin, Ethylendiamin, Dicyclohexylamin, N-Cyclohexyl-N-3-aminopropylamin, 1-Aminoadamantan oder 1-Amino-3.5-dimethyladamantan,
- ggf. durch eine oder mehrere Niederalkyl-(= C₁ bis C₄-Alkyl) oder Hydroxyl-Reste substituierte cyclische oder heterocyclische Amine wie Pyrrolidin, Piperidin, Morpholin, Piperazin, N-Methylpiperazin oder N-Methylhomopiperazin,
- ggf. substituierte aromatische, heteroaromatische, arylaliphatische oder heteroarylaliphatische Amine wie Benzylamin, 3.4.5-Trimethoxybenzylamin, Veratrylamin, Phenethylamin, Homoveratrylamin, N-Methylhomoveratrylamin, 4-Aminopyridin, Tacrin und Analoga, Imipramin, Desipramin, Selegilin, Nicotin, Pindolol,
- Aminosäureester und -amide wie Methyl-, Ethyl-, Propyloder Isopropylester und Amide von Glycin, Alanin, Phenylalanin, Leucin, Isoleucin, Methionin, Prolin, Valin, Sarcosin, Pipecolinsäure,
- sowie basische Aminosäuren wie Lysin oder Arginin oder deren Amide.

Besonders geeignete Basen sind solche, die selber Wirkstoffe sind und den gleichen medizinischen Indikationsbereich wie das Hyperforin besitzen oder dessen therapeutische Verwendung unterstützen. Infrage kommen hierzu vor allem basische Wirkstoffe mit den Indikationen:
- **Alzheimersche Krankheit (AD)**, z. B. Acetylcholinesterase-Inhibitoren (z. B. Amiridin, Donezepil, Ensaculin, Eptastigmin, Galanthamin, Huperzin A, 7-Methoxytacrin, Physostigmin, SDZ-ENA-713 (Exelon), SM-10888, Suronacrin, Tacrin, Velnacrin), cholinerge Aktivatoren, NMDA-Antagonisten (z. B. Memantin), Glutaminrezeptor-Antagonisten, Serotonin-Agonisten und **Antagonisten** (z. B. Adatanserin), Monaminoxidase-Inhibitoren (z. B. Tranylcypromin, Selegelin), PKC-Aktivatoren und α-Sekretasehemmer, Tyrosinkinase-Antagonisten, Muscarin-Agonisten (z. B. Arecolin, BIBN 99, Itamelin, Milamelin, Talsaclidin, Xanomelin, YM796),
- **Antidepressiva,** z.B. Amitryptilin, Dibenzepin, Desipramin, Desitryptilin, Dosulepin, Doxepin, Clomipramin, Fluoxetin, Fluvoxamin, Imipramin, Lofepramin, Maprotilin, Moclebemid, Mianserin, Nortriptylin, Opipramol, Paroxetin, Tranylcypromin, Trazodon, Trimipramin, Viloxazin
- **Anxiolytika**, z. B. Chlorprothixen, Dixyrazin, Fluphenazin, Levomepromazin, Melperon, Perphenazin, Promazin, Promethazin, Pritiphendyl, Sulpirid, Tandospiron, Thioridazin, Trifluoperazin, Zuclopentixol,
- **Calcium-Antagonisten** (mit basischer Seitenkette) z. B. Amlodipin, Azelnidipin, Barnidipin, Benidipin, Cronidipin, Edrecolomab (AE0047), Efonidipin, Elgodipin, Lercanidipin, Manidipin, Nicardipin, Palonidipin, Verapamil,
- **Dyspepsie-Therapeutika und Prokinetika**, z. B. Cisaprid, Metoclopramid, Renzaprid (5-HT₄ Agonisten),
- **β-Rezeptorenblocker**, z. B. Atenolol, Alprenolol, Carazolol, Propranolol, Labetalol, Mepindolol, Metoprolol, Oxprenolol, Penbutolol, Pindolol, Bupranolol, Bunitrolol, Metipranolol, Nadolol,
- **Nootropica**, z. B. Lomerizin, Nebracetam, Pramiracetam, SNK-882.

Salze des Hyperforins und Adhyperforins mit derartigen basischen Wirkstoffen bilden einen besonders innovativen Teilaspekt der vorliegenden Erfindung, da sie nicht nur die Stabilität des therapeutisch wirksamen Hyperforins erhöhen, sondern zusätzlich infolge ihrer eigenen therapeutischen Wirkung eine besonders sinnvolle Kombination sich gegenseitig verstärkender oder unterstützender Wirkprinzipien erlauben.

### Herstellung der erfindungsgemässen Salze:

Die erfindungsgemässen Salze können auf verschiedenen Wegen hergestellt werden. Bei der folgenden Methoden-Erläuterung sind mit Begriff "Hyperforin" stets auch das Homologe Adhyperforin sowie auch Gemische aus beiden Substanzen gemeint. "Nieder-" bedeutet immer "C₁ bis C₄ -".

**Methode A:** Hyperforin wird in einem Niederalkanol (z. B. Methanol, Ethanol, Propanol oder Isopropanol) vorzugsweise unter Schutzgas und Lichtausschluss gelöst, mit der Lösung einer equimolaren Menge Alkalimetall-Hydroxid oder -Nieder-alkanolat (z. B. Natrium-Methylat oder -Ethylat) in einem der zuvor genannten Niederalkanole versetzt, die Lösung evaporiert, mit Wasser aufgenommen und lyophilisiert. Man erhält stabile farblose bis cremefarbene pulverförmige Alkalisalze des Hyperforins. Alternativ kann das Hyperforin auch direkt in der Lösung des Alkalimetall-Niederalkanoats aufgelöst und wie oben aufgearbeitet werden.

**Methode B:** Hyperforin wird in einem aprotischen Lösungsmittel, ausgewählt aus der Gruppe der apolaren C1-C10-Alkane und -Cycloalkane, z. B. Pentan, Hexan, Heptan, Octan, Isooctan oder Cyclohexan, ggf. unter Zugabe geringer Mengen eines niederen Alkanols (z. B. Methanol, Ethanol oder Isopropanol) vorzugsweise unter Schutzgas und Lichtausschluss gelöst, diese Lösung mit der equimolaren Menge der basischen Komponente B oder einer Lösung derselben in einem der zuvor angegebenen Lösungsmittel oder in einem Niederhalogenalkan, z. B. Dichlormethan oder Chloroform, oder in einem niederen Ether, z. B. Diethylether, Diisopropylether, tert.-Butylmethylether oder Tetrahydrofuran, oder in einem niederen Keton, z. B. Aceton oder Methylethylketon, versetzt, die Mischung ggf. konzentriert, das ausfallende Salz abgetrennt, ggf. umkristallisiert und im Vakuum getrocknet. Man erhält kristalline oder amorphe pulverförmige Ammoniumsalze des Hyperforins.
Falls die Base B mehrere zur Salzbildung fähige basische Zentren enthält, können, falls gewünscht, entsprechend geringere Mengen z. B. 1/2-molare oder 1/3-molare Mengen, an Base B eingesetzt werden, sodass das Verhältnis Hyperforin/Base = m/p beträgt.

**Methode C:** Hyperforin wird in einem Niederalkanol (z. B. Methanol, Ethanol oder Isopropanol), vorzugsweise unter Schutzgas und Lichtausschluss, gelöst, diese Lösung mit der equimolaren Menge der basischen Komponente B oder einer Lösung derselben in einem der zuvor angegebenen Lösungsmittel versetzt, die Mischung evaporiert, in Wasser aufgenommen und lyophilisiert. Man erhält kristalline oder amorphe pulverförmige Ammoniumsalze des Hyperforins.

**Methode D:** Hyperforin wird in einem Niederalkanol (z. B. Methanol, Ethanol oder Isopropanol), vorzugsweise unter Schutzgas und Lichtausschluss, gelöst, diese Lösung mit der Lösung einer equimolaren Menge der basischen Komponente B in Wasser versetzt, der niedere Alkohol weitgehend im Vakuum abdestilliert und das verbleibende wässrige Gemisch ggf. nach Zusatz von Wasser lyopholisiert. Das erhaltene pulverförmige Salz wird ggf. aus einem niederen Alkohol, Alkohol/Wasser-Gemisch oder aus einem niederen Ester umkristallisiert.

### Verfahren zur Anreicherung bzw. Reinisolierung von Hyperforin und Adhyperforin aus Johanniskraut-Extrakten:

Bisher war die Reingewinnung von Hyperforin aus Johanniskraut-Extrakt nur über sehr aufwendige chromatographische Methoden möglich, die zudem in technisch unzumutbarer Weise durch die hohe Instabilität des Hyperforins und seines Homologen gegenüber Licht und Luftsauerstoff erschwert wurden (P. Maisenbacher, Universität Tübingen, Diss. 1991. R. Burgdörfer, Universität Marburg, Diss. 1987).

Es wurde nun eine überraschend einfache und kostensparende Lösung dieses technischen Problems gefunden, indem man Johanniskraut-Extrakte, z.B. einen CO₂-Extrakt mit 20-80% Hyperforin/ Adhyperforin-Gehalt, in einem geeigneten Lösungsmittel, ausgewählt aus der Reihe apolarer C1-C10-Alkane und -Cycloalkane, z. B. Pentan, Hexan, Heptan, Octan, Isooctan oder Cyclohexan, ggf. unter Zugabe geringer Mengen eines niederen Alkanols (z. B. Methanol, Ethanol oder Isopropanol), vorzugsweise unter Schutzgas und Lichtausschluss, löst, diese Lösung mit der mindestens equimolaren Menge der basischen Komponente **B** oder einer Lösung derselben in einem der zuvor angegebenen Lösungsmittel oder in einem Niederhalogenalkan, z. B. Dichlormethan oder Chloroform, oder in einem niederen Ether, z. B. Diethylether, Diisopropylether, tert.-Butylmethylether oder Tetrahydrofuran, oder in einem niederen Keton, z. B. Aceton oder Methylethylketon, versetzt, die Mischung ggf. konzentriert, das ausfallende Salz abtrennt, ggf. umfällt und/oder umkristallisiert und im Vakuum trocknet. Man erhält kristalline oder amorphe pulverförmige Ammoniumsalze des Hyperforins / Adhyperforins bzw. eines Gemisches derselben.

Für dieses Verfahren besonders geeignete salzbildende Amine sind cycloaliphatische (z. B. Dicyclohexylamin), araliphatische (z.B. Benzylamin und dessen methoxy-substituierte Derivate), heterocyclische oder heteroaromatische Amine (z. B. 4-Aminopyridin).

Hyperforin / Adhyperforin können aus den kristallinen und lagerstabilen Salzen, durch Ansäuern, vorzugsweise mit einer organischen Säure (z. B. Citronensäure oder Weinsäure), und nachfolgender Verteilung zwischen einem der angeführten Lösungsmittel und Wasser bequem in reiner Form erhalten und als solche weiterverwendet bzw. in andere gewünschte Salze übergeführt werden. Hierzu wird das Hyperforin-Salz unter Schutzgas-Atmosphäre und Lichtauschluss in dem gewünschten Lösungsmittel (z. B. Methyltert.butylether oder Ethylacetat) gelöst oder suspendiert, mit der mindestens äquimolaren Menge der in Wasser gelösten Säure versetzt, bis zur völligen Auflösung gerührt, die Wasserphase abgetrennt und die organische Phase nach Waschen mit Wasser schonend evaporiert.

### Arzneimittel:

Die vorliegende Erfindung betrifft weiterhin Arzneimittel, die neben nichttoxischen, inerten pharmazeutisch geeigneten Trägerstoffen eines oder mehrere der erfindungsgemässen Hyperforinund/oder Adhyperforin-Salze enthalten oder die aus einem oder mehreren der erfindungsgemässen Hyperforin- und/oder Adhyperforin-Salze bestehen, sowie Verfahren zur Herstellung dieser Arzneimittel.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Geeignete feste oder flüssige galenische Zubereitungsformen der erfindungsgemässen Arzneimittel sind z. B. Tabletten, Kapseln, Dragees, Suppositorien, Sirupe, Emulsionen, Suspensionen, Tropfen oder injizierbare Lösungen sowie Präparate mit protrahierter Wirkstoff-Freigabe.

Als Trägerstoffe bzw. Verdünnungsmittel seien z.B. verschiedene Zucker- oder Stärkearten, Cellulosederivate, Magnesiumcarbonat, Gelatine, tierische und pflanzliche Oele, Polyethylenglykole, Wasser oder andere physiologisch verträgliche Lösungsmittel sowie wasserhaltige Puffermittel, die durch Zusatz von Salzen oder Glukose isotonisch gemacht werden können, genannt. Ausserdem können gegebenenfalls oberflächenaktive Mittel, Farb- und Geschmacksstoffe, Stabilisatoren und Konservierungsmittel als weitere Zusatzstoffe in den erfindungsgemässen Arzneimitteln Verwendung finden.

Die therapeutisch wirksamen Verbindungen sind in den oben angeführten Arzneimitteln vorzugsweise in einer Konzentration von etwa 0.5 bis 95 % der Gesamtmischung vorhanden. Die Herstellung der Arzneimittel erfolgt nach dem Fachmann geläufigen Methoden, z.B. durch Mischen des oder der Wirkstoffe mit den Träger- und Zusatzstoffen und Weiterverarbeitung zu der gewünschten galenischen Form.

Die Erfindung betrifft des weiteren auch die Verwendung der erfindungsgemässen Wirkstoffe sowie der aus ihnen hergestellten Arzneimittel in der Humanmedizin zur Therapie oder zur Prophylaxe der Alzheimerschen Krankheit.

Schließlich betrifft die Erfindung die Verwendung des als Inhaltsstoff von therapeutisch genutzten Extrakten bekannten Adhyper-forins und von Gemischen aus Hyperforin und Adhyperforin, gegebenenfalls zusammen mit einem pharmazeutisch verträglichen Träger oder Verdünnungsmittel, in der Humanmedizin zur Therapie oder zur Prophylaxe der Alzheimerschen Krankheit (2. medizinische Indikation).

Die erfindungsgemässen Wirkstoffe oder Arzneimittel können oral, parenteral, intravenös und/oder rektal appliziert werden. Die Dosierung der Wirkstoffe in der Humanmedizin erfolgt vorzugs-weise in Gesamtmengen von 0.01 bis 10, insbesondere 0.05 bis 5 mg/kg Körpergewicht je 24 Stunden, gegebenenfalls in Form mehrerer Einzelgaben. Die Gesamtmenge wird in 1 bis 5, vorzugs-weise in 1 bis 3 Einzeldosen verabreicht. Die Festlegung und die zeitliche Abfolge der Dosierung sowie die Wahl der geeigneten Applikationsart kann durch jeden Fachmann aufgrund seines Fachwissens leicht erfolgen.

Folgende Beispiele sollen die Erfindung erläutern, ohne sie in ihrem Umfang einzuschränken.

**Allgemeines:** Schmelzpunkte wurden mit den Geräten Elektrothermal® oder B-545 (Büchi) gemessen, IR-Spektren wurden mit dem IR-Spektrometer IFS 28 (Bruker) an KBr-Presslingen, NMR-Spektren mit dem AC 200 oder Avance 200 (Bruker) in D₄-Methanol aufgenommen (200 MHz für ¹H und 50 MHz für ¹³C; δ-Werte in ppm).
HPLC-Bestimmungen wurden mit dem Dynamax PDA-1 von Rainin, Voburn, MA, USA durchgeführt.
HPLC-Bedingungen (isokratisch): Adsorbens: Eurospher 100-C18, 10 µm. Eluens: Acetonitril/Wasser/Phosphorsäure = 85/15/0.3 (Vol/Vol). Detektion: 273 nm. Referenzstandard: Hyperforin-Dicyclohexylamin-Salz gemäss Beispiel 2b = 100 %. Assay:
Gehaltsangaben in area-% bezüglich Referenzstandard. Bei Gehaltsangaben für Hyperforin (Adhyperforin) in deren Salzen sind die Werte stets in Prozent des als 100 % gesetzten stöchiometrischen Anteils des Hyperforins (Adhyperforins) angegeben.
Lösungsmittel: Methanol (p.a. oder reinst), Methyltert.butylether (> 99 %), Isopropanol (> 99 %) und Wasser (bidestilliert) werden vor Gebrauch entgast.
Arbeitsbedingungen: unter Rotlicht oder in abgedunkelten Apparaturen unter Schutzgas (Stickstoff oder Argon).
Elementaranalysen: lyophilisierte Produkte enthalten gelegentlich noch Restwasser. Sie werden als Hydrate berechnet. Abkürzungen: MTBE = Methyl-tert.butylether. "98H/I" = n-Heptan / Isopropanol 98/2 (Vol/Vol). Fp = Schmelzpunkt.
wfr = wasserfrei. Th =Theorie, ss = sehr stark.

### Beispiel Nr. 1a (Methode A): Natrium-Salz des Hyperforins.

23 mg (1 mmol) Natrium werden in 50 ml wfr. Methanol gelöst. In dieser Lösung werden 536 mg (1 mmol) Hyperforin unter Rühren aufgelöst und die Lösung evaporiert. Der Rückstand wird in 100 ml Wasser gelöst und lyophilisiert. Ausbeute: 556 mg helles Pulver (99.6 % der Th.). Fp: Zersetzung ab 170°C. IR: 1420-1500 cm⁻¹ (ss).

### Beispiel Nr. 1b (Methode A): Natrium-Salz des Hyperforins.

728.5 mg (1.31 mmol) 96.5 %-iges Hyperforin werden in 80 ml Methanol gelöst, mit 5.1 ml (1.32 mmol) 0.259-molarer methanolischer Natriumhydroxid-Lösung versetzt, kurz stehen gelassen und die Lösung bei 50°C evaporiert. Der Rückstand wird in Wasser gelöst und lyophilisiert. Ausbeute: 746.7 mg (1.33 mmol) weisses Pulver (101 % der Th.). Fp: Sintern zwischen 90°C und 110°C, Zersetzung bei 170°C. IR: 1499 cm⁻¹ (ss, breit). ¹H-NMR: keine Verunreinigungen ausser ca. 1.4 Mol% (0.08 Gew.%) Methanol sichtbar. C₃₅H₅₁NaO₄ (558.78). Berechnet/Gefunden: C (75.23/71.61), H (9.20/8.87); Na (4.11/4.6). Hyperforin-Gehalt (HPLC): 86.5 %.

### Beispiel Nr. 2a (Methode B): N.N-Dicyclohexylamin-Salz des Hyperforins.

1 g (1.86 mmol) Hyperforin wird in 50 ml n-Pentan/Methanol 98/2 Vol/Vol gelöst, darauf mit 445 µl (2.33 mmol) Dicyclohexylamin versetzt und 10 Stdn. bei 4°C stehen gelassen. Ausgefallenes Produkt wird über eine Glasfilterfritte abgesaugt, mit Pentan gewaschen und bei Raumtemperatur im Vakuum getrocknet. Ausbeute: 652 mg weisses Pulver (48.7 % der Th.). Fp: 157.2°C. IR: 1473, 1489 cm⁻¹ (ss). C₄₇H₇₅NO₄ (718.13). Berechnet/Gefunden: C (78.61/76.91), H (10.53/10.42), N (1.95/1.70). NMR: ausser den Signalen des Hyperforins sind folgende Signale des Dicyclohexylamins sichtbar: ¹H-NMR: 3.15 (m; 2 H; 1-C*H*) und (z.T. überlagert) 1.85-2.08 (m, 2- und 6-C*H*₂), 1.42 (quint, 3-, 4- und 5-C*H*₂). Quotient Dicyclohexylamin/Hyperforin = 1/1. Keine Verunreinigungen sichtbar. ¹³C-NMR: 54.65 (1-*C*H), 30.76 (2- und 6-*C*H₂), 26.06 (4-CH₂) und 25.64 (3- und 5-*C*H₂).

Die Substanz wird aus Pentan/Methanol umkristallisiert. Fp: 163.9°C. C₄₇H₇₅NO₄ (718.13). Berechnet/Gefunden: C (78.61/78.92), H (10.53/10.44), N (1.95/1.79). Hyperforin-Gehalt (HPLC): 100 %.

### Beispiel Nr. 2b (Methode B): N.N-Dicyclohexylamin-Salz des Hyperforins.

1.547 g (2.82 mmol) 98 %-iges Hyperforin werden in 60 ml n-Heptan/Isopropanol 98/2 Vol/Vol gelöst, darauf mit 600 µl (3.0 mmol) Dicyclohexylamin versetzt und 18 Stdn. unter N₂ bei Raumtemperatur stehen gelassen. Ausgefallenes Produkt wird über eine Glasfilterfritte abgesaugt, mit Heptan gewaschen und 8 Stdn. bei Raumtemperatur im Vakuum getrocknet. Ausbeute: 1.767 g (2.46 mmol) weisses Pulver (87 % der Th.). Fp: 159.7°C. IR: 1473, 1489 cm⁻¹ (ss). ¹H-NMR: entspricht dem ¹H-NMR-Spektrum von Beispiel 2a. C₄₇H₇₅NO₄ (718.13). Berechnet/Gefunden: C (78.61/78.59), H (10.53/10.66), N (1.95/1.87). Hyperforin-Gehalt (Titration mit HClO₄ ): 100 %.

### Beispiel Nr. 3a (Methode B): 3.4.5-Trimethoxbenzylamin-Salz des Hyperforins.

57.8 g (0.1 mmol) 93.5 %-iges Hyperforin werden in 2 ml n-Pentan gelöst und sofort mit 50 µl einer 2M-Lösung von 3.4.5-Trimethoxybenzylamin in MTBE versetzt. Der farblose Niederschlag wird abgesaugt, mit Pentan gewaschen und bei Raumtemperatur im Vakuum getrocknet. Ausbeute: 10 mg weisses Kristallisat (14 % der. Th.). IR: 1480 cm⁻¹ (ss). C₄₅H₆₇NO₇ (734.04).

### Beispiel Nr. 3b (Methode C): 3.4.5-Trimethoxbenzylamin-Salz des Hyperforins.

58.3 mg (0.1 mmol) 92.7 %-iges Hyperforin werden in 2 ml wfr. Methanol gelöst. mit 19.7 mg (0.1 mmol) frisch destilliertem 3.4.5-Trimethoxybenzylamin versetzt, mit 2 ml Methanol verdünnt und bei 50°C evaporiert. Das Evaporat wird in 20 ml Wasser aufgenommen und lyophilisiert. Ausbeute: 70.4 mg weisses Pulver (96 % der Th.). Fp: 126-33°C. IR: 1484 cm⁻¹ (ss). C₄₅H₆₇NO₇ (734.04). Hyperforin-Gehalt (HPLC): 87.2%.

### Beispiel Nr. 4 (Methode D): L-Arginin-Salz des Hyperforins.

58.3 mg (0.1 mmol) 92.7 %-iges Hyperforin werden in 2 ml wfr. Methanol gelöst, mit der Lösung von 17.4 mg (0.1 mmol) L-Arginin in 0.5 ml bidest. Wasser und 15 ml Methanol versetzt und bei 50°C im Vakuum konzentriert. Der Rückstand wird mit 10 ml Wasser verdünnt und lyophilisiert. Ausbeute: 58.1 mg weisses Pulver (81.7 % der Th.). Fp: Sintern ab 110°C, Zersetzung bei 145°C.
IR: 1486 cm⁻¹ (ss). C₄₁H₆₆NO₆ (711.01). Hyperforin-Gehalt
(HPLC): 82.7 %.

### Beispiel Nr. 5 (Methode B): Pyrrolidin-Salz des Hyperforins.

578 mg (1.0 mmol) 93.5 %-iges Hyperforin werden in 15 ml n-Pentan gelöst, darauf mit 85 µl (1.0 mmol) Pyrrolidin versetzt und 24 Stdn. bei Raumtemperatur (keine Kristallisation) und 24 h bei -20°C stehen gelassen (Produkt fällt ölig aus). Das Gemisch wird evaporiert, das Evaporat in Wasser/Methanol gelöst und lyophilisiert. Ausbeute: 576 mg (0.947 mmol) farbloses Pulver (94.7 % der Th.). IR: 1489 cm⁻¹ (ss). C₃₉H₆₁NO₄ (607.93). Hyperforin-Gehalt (HPLC): 93.7 %.

### Beispiel Nr. 6 (Methode D): Ethylendiamin-Salz mit 2 Mol Hyperforin.

58.3 mg (0.1 mmol) 92.7 %-iges Hyperforin werden in 2 ml Methanol gelöst, mit 50 µl einer 1M-Lösung von Ethylendiamin in Wasser und 2 ml Methanol versetzt und bei 50°C im Vakuum konzentriert. Der Rückstand wird mit 20 ml Wasser verdünnt und lyophilisiert. Ausbeute: 57.1 mg weisses Pulver (100.7 % der Th.). Fp: (Sintern ab 40°C) 50-2°C. IR: 1480 cm⁻¹ (ss).
C₇₂H₁₁₂N₂O₈ (1133.70). Hyperforin-Gehalt (HPLC): 83.1 %.

### Beispiele Nr. 7 bis 20

Weitere Beispiele für erfindungsgemässe Salze des Hyperforins sind in der nachstehende Tabelle Nr. I aufgeführt. Die Salze liegen nach dem Lyophilisieren als farblose bis cremefarbene Pulver vor. Ihre Konstitution wird NMR- und IR-spektroskopisch bestätigt.

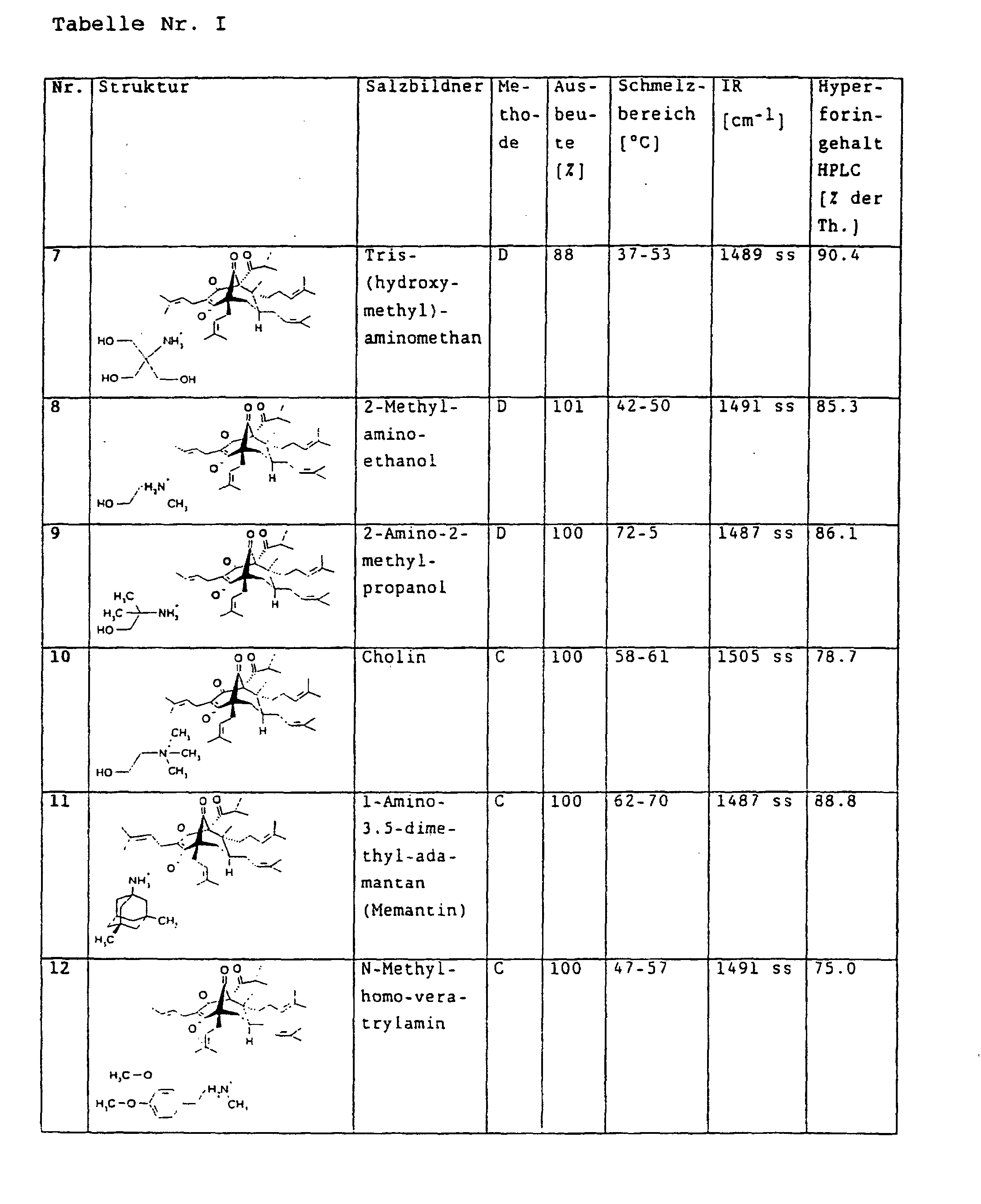

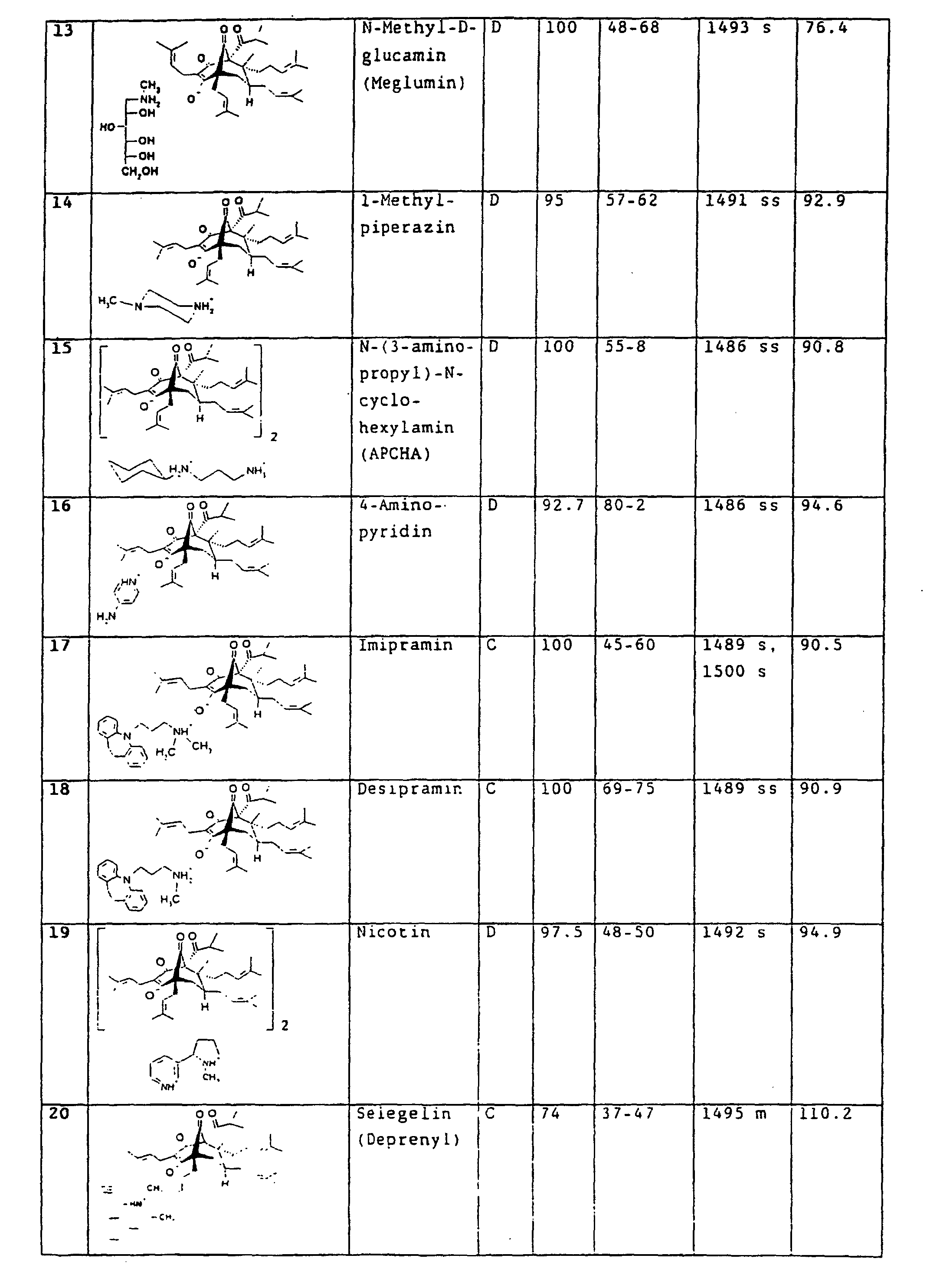

### Beispiel Nr. 21: Isolierung von Hyperforin/Adhyperforin aus einem Hypericum-CO₂-Extrakt (Gehalt: 32.1 % Hyperforin, 6.8 % Adhyperforin) durch Fällung als N,N-Dicyclohexylamin-Salz und Umkristallisation.

a) 200 g Extrakt (145 mmol Hyperforin + Adhyperforin) werden mit 2.8 L n-Heptan/Isopropanol 98/2 ("98H/I") bei 40°C im Rotationskolben extrahiert, 200 g wfr. Natriumsulfat zugesetzt, 30 min gerührt, vom Ungelösten abfiltriert (Super Seitz 1500 Filterplatte) und mit 200 ml "98H/I" nachgewaschen. Unter Rühren werden dem Filtrat 29 g (160 mmol) Dicyclohexylamin zugetropft und die Mischung 16 h bei 20°C stehen gelassen. Das Kristallisat wird abgesaugt, mit "98H/I" gewaschen und im Vakuum getrocknet (K1: 55.76 g). Die Mutterlauge wird auf 1/3 Vol eingeengt, 16 h bei 20°C gelagert und auf 4°C gekühlt. Vom Kristallisat wird der Überstand abdekantiert, das Kristallisat mit "98H/I" gewaschen und getrocknet (K2: 37.95 g).
   Das Rohkristallisat (K1 + K2: 93.71 g; HPLC-Gehalt: 67.2 % Hyperforin, 14.1 % Adhyperforin) wird unter Erwärmen in 400 ml Methanol (reinst) gelöst, 4 h bei 4°C gelagert, ausgefallenes wachsartiges Material abgesaugt, das Filtrat eingeengt, in 200 ml MTBE gelöst, mit 300 ml n-Pentan versetzt, 16 h bei 20°C gelagert und auf 4°C abgekühlt. Das Kristallisat wird abgesaugt, 2 x mit kaltem MTBE/Pentan gewaschen, im Vakuum (ca. 20 hPa) vorgetrocknet und bei 60°C / 0.1 hPa nachgetrocknet [46.37 g K3; Fp: 161.6 - 162.0°C. HPLC-Gehalt: 84.9 % Hyperforin, 17.5 % Adhyperforin]. Die Mutterlauge wird auf 2/3 Vol konzentriert, w. o. gelagert, das Kristallisat abgesaugt, w. o. gewaschen und getrocknet [30.92 g K4]; Fp: 158.8 - 159.2°C. HPLC-Gehalt: 78.9 % Hyperforin, 19.2 % Adhyperforin].
   Gesamtausbeute: 77.29 g Dicyclohexylamin-Salz von Hyperforin/Adhyperforin (ca. 82/18) = ca. 74 %.
b) 1000 g Extrakt (725 mmol Hyperforin + Adhyperforin) werden in 15 L Methanol 15 min bei 22-29 °C mit einem Ultra-Turax dispergiert, 17 h bei 4 °C gelagert und ausgefallene Wachse über einen Seitz-Supra-2600 Einschichtfilter abfiltriert. Der Filterkuchen wird mit 1 L Methanol gewaschen und die vereinten Filtrace bei 40°C im Vakuum auf ca. 1/3 Vol konzentriert. Das auf 20 °C abgekühlte Konzentrat wird mit Heptan gesättigt, mit 3 x 2 L methanolgesättigtem Heptan extrahiert und die vereinten Extrakte mit 2 x 500 ml heptangesättigtem Methanol reextrahiert. Die vereinten Methanolextrakte werden bei 40 °C evaporiert, danach in 8 L "98H/I" bei 40 °C unter Rotation gelöst, auf 20 °C gekühlt, unter Rühren, Lichtschutz und Argon mit 159 ml (798 mmol) Dicyclohexylamin versetzt und die sofort kristallisierende Mischung 16 h bei 4 °C gelagert. Das Kristallisat wird abgesaugt, mit kaltem "98H/I" gewaschen und im Vakuum getrocknet. Das Trockenprodukt (425 g) wird in 1.5 L MTBE suspendiert, 5 min bei 40 °C gerührt, nach Abkühlen auf 20 °C das Kristallisat abgesaugt, mit kaltem MTBE gewaschen und 24 h bei 20 °C / 10 hPa getrocknet. Ausbeute: 355.0 g (494 mmol) = 68.2 %. Fp: 161.0°C. HPLC-Gehalt: 82.44 % Hyperforin, 17.39 % Adhyperforin, zusammen 99.83 %.

### Beispiel Nr. 22: Salz von Adhyperforin mit Dicyclohexylamin.

Aus einem Anteil des Hyperforin/ Adhyperforin-Dicyclohexylamin-Salzes (K4 des Beispiels 21) wird mittels präparativer HPLC an RP-18 Adsorbens das Adhyperforin isoliert (HPLC-Gehalt: 93.8 %). Hiervon werden 34 mg (62 µmol) in 2 ml "98H/I" unter N₂ und Lichtausschluss gelöst, 12.5 µl (63 µmol) Dicyclohexylamin zudotiert, nach 18 h das Kristallisat abgesaugt, mit kaltem "98H/I" gewaschen und 18 h im Vakuum getrocknet. Ausbeute: 12.5 mg (17 µmol) = 27 %. HPLC-Gehalt: 91.2 % Adhyperforin.

**Beispiel Nr. 23:** Freisetzung von Hyperforin/Adhyperforin aus einem Dicyclohexylamin-Salz von Hyperforin/Adhyperforin (HPLC-Gehalt: 86 % Hyperforin, 15 % Adhyperforin). Unter Rühren werden 718 mg (1.0 mmol) Hyperforin/Adhyperforin-Dicyclohexylamin-Salz in 60 ml MTBE suspendiert, 10 ml 1-molarer wässriger Citronensäure zugegeben, 30 min gerührt, die MTBE-Phase abgetrennt, 3 x mit Wasser gewaschen, über Natriumsulfat getrocknet und evaporiert. Das farblose Öl wird bei 20 °C / 0.1 hPa getrocknet. Ausbeute: 527.6 mg (0.983 mmol) = 98.3 %. HPLC-Gehalt: 86.8 % Hyperforin, 14.9 % Adhyperforin.

**Beispiel Nr. 24:** Kalium-Salz von Hyperforin/Adhyperforin. 537 mg (1.0 mmol) Hyperforin/Adhyperforin-9/1-Gemisch werden in 100 ml Methanol gelöst, mit 10 ml einer 0.1M-KOH in Methanol versetzt und evaporiert. Der Rückstand wird in 80 ml Wasser aufgenommen und lyophilisiert. Ausbeute: 590.6 mg (99.6 mmol) = 99.6 % farbloses Pulver. Fp: 110-120 °C (Sintern). HPLC-Gehalt: 78.7 % Hyperforin, 8.8 % Adhyperforin. IR: 1499.5 cm⁻¹ (ss). C₃₅H₅₁KO₄ x H₂O(592.91).

**Beispiel Nr. 25:** Lithium-Salz von Hyperforin/Adhyperforin. 2.68 g (5.0 mmol) Hyperforin/Adhyperforin-5/1-Gemisch werden in 50 ml Methanol gelöst, mit der Lösung von 0.210 (5.0 mmol) Lithiumhydroxid-Hydrat in 20 ml Methanol versetzt, das Gemisch bei 40 °C evaporiert, der Rückstand in 30 ml Wasser gelöst und lyophilisiert. Ausbeute: 2.735 g (4.88 mmol) = 97.5 % farbloses Pulver. Schmelzbereich: 80-93 °C. HPLC-Gehalt: 80.2 % Hyperforin, 15.0 % Adhyperforin; zusammen 95.2 %. IR: 1493.1 cm⁻¹ (ss). C₃₅H₅₁LiO₄ x H₂O(560.74).

**Beispiel Nr. 26:** L-Lysin-Salz von Hyperforin/Adhyperforin. 2.68 g (5.0 mmol) Hyperforin/Adhyperforin-5/1-Gemisch werden in 50 ml Methanol gelöst, unter Rühren mit der Lösung von 0.731 g (5.0 mmol) L-Lysin in 10 ml Wasser versetzt und das klare Gemisch bei 40 °C evaporiert. Der Rückstand wird nach Zugabe von 50 ml Wasser lyophilisiert. Ausbeute: 3.35 g (4.78 mmol) = 95.6 % farbloses Pulver. Schmelzbereich: 74-98 °C. HPLC-Gehalt: 83.25 % Hyperforin, 16.98 % Adhyperforin, zusammen 100.2 %. IR: 1483 cm⁻ ¹ (ss). C₄₁H₆₆N₂O₆ x H₂O (701.07).

**Beispiel Nr. 27:** Pindolol-Salz von Hyperforin/Adhyperforin. 2.68 g (5.0 mmol) Hyperforin/Adhyperforin-8/1-Gemisch und 1.24 g (5.0 mmol) Pindolol werden in 50 ml Methanol gelöst und bei 40 °C evaporiert. Der Rückstand wird nach Zugabe von 50 ml Wasser lyophilisiert. Ausbeute: 4.03 g (5.1 mmol) = 102 % weisser Schaum. Schmelzbereich: 65-75 °C. HPLC-Gehalt: 91.2 % Hyperforin, 11.7 % Adhyperforin, zusammen 102.9 %. C₄₉H₇₂N₂O₆ (701.07).

**Beispiel Nr. 28:** Pyrrolidin-Salz von Hyperforin/Adhyperforin. 531 mg (0.99 mmol) Hyperforin/Adhyperforin-5/1-Gemisch wird unter Lichtausschluss in 10 mL Methanol gelöst, darauf mit 83.5 µl (0.98 mmol) Pyrrolidin versetzt und am Rotverdampfer konzentriert. Das Konzentrat wird in 70 ml Wasser aufgenommen und lyophilisiert. Ausbeute: 570.6 mg (0.938 mmol) = 95%. Schmelzbereich: 50-70 °C. HPLC-Gehalt: 79.5 % Hyperforin, 16.4 % Adhyperforin, zusammen 95.9 %. IR: 1489 cm⁻¹ (ss).
C₃₉H₆₁NO₄ (607.93).

**Beispiel Nr. 29:** Desipramin-Salz von Hyperforin/Adhyperforin. 2.68 g (5.0 mmol) Hyperforin/Adhyperforin-5/1-Gemisch werden unter N₂ und Lichtausschluss in MTBE/Pentan 9/1 in der Wärme unter Zugabe einiger Tropfen Methanol gelöst und dann bei -20 °C kristallisiert. Das Kristallisat wird abgesaugt, mit eiskaltem Pentan/MTBE 9/1 gewaschen und bei RT / 0.1 hPa getrocknet. Ausbeute: 4.03 g (5.0 mmol) = 100 % weisses Kristallpulver. Fp: 154.8-155.3 °C. HPLC-Gehalt: 82.4 % Hyperforin, 16.3 % Adhyperforin, zusammen 98.7 %. IR: 1489 cm⁻¹ (ss). C₅₃H₇₄N₂ O₄
(803.19).

**Beispiel Nr. 30:** Natrium-Salz von Hyperforin/Adhyperforin. 5.94 g (11.1 mmol) Hyperforin/Adhyperforin-9/1-Gemisch werden unter N₂ und Lichtausschluss in 50 ml Methanol gelöst, mit 10.7 ml 1M-NaOH versetzt und das Methanol am Rotationsverdampfer bei 40 °C im Vakuum abgezogen. Der Rückstand wird mit 50 ml Wasser aufgenommen und lyophilisiert. Ausbeute: 6.2 g (11.1 mmol) = 97.2 % weisses Pulver. Schmelzbereich: 109-128 °C. HPLC-Gehalt: 91.3 % Hyperforin, 9.5 % Adhyperforin; zusammen 100.8 % der Theorie. IR: 1499 cm⁻¹ (ss). C₃₅H₅₁NaO₄ x H₂O.(576.80).

**Beispiel Nr. 31:** L-Arginin-Salz von Hyperforin/Adhyperforin. 2.68 g (5.0 mmol) Hyperforin/Adhyperforin-5/1-Gemisch werden unter N2 und Lichtausschluss in 50 ml Methanol gelöst, mit der Lösung von 0.87 g (1.0 mmol) L-Arginin in 10 ml Wasser versetzt und bei 50°C im Vakuum konzentriert. Der Rückstand wird mit 50 ml Wasser verdünnt und lyophilisiert. Ausbeute: 3.59 g (4.92 mmol) = 98.5 % weisses Pulver. Schmelzbereich: 115-133°C. IR: 1486 cm⁻¹ (ss). HPLC-Gehalt: 82.6 % Hyperforin, 17.0 % Adhyperforin; zusammen 99.6 %. C₄₁H₆₆NO₆ x H₂O (729.02).

**Beispiel Nr. 32:** Stabilität von Hyperforin-Salzen.
Proben von Hyperforinsalzen werden in mit einem Schnappdeckelverschluss versehenen Braunglasflaschen ohne Schutzgas bei Raumtemperatur gelagert und der Gehalt an Hyperforin (Beispiele 1 bis 20) bzw. an Hyperforin + Adhyperforin (Beispiele 21 bis 31) mittels HPLC (area%) zu den Lagerzeiten 0, 1 Woche, 4 Wochen und 12 Wochen bestimmt. Die Änderung des Gehalts an Hyperforin (+ Adhyperforin) sind in Tabelle Nr. II dargestellt.

**Tabelle Nr. II:**

| Stabilität von Hyperforin-Salzen | | | | | |
|---|---|---|---|---|---|
| Änderung des Gehalts an Hyperforin (% des stöchiometrischen Gehalts) | | | | | |
| Substanz gemäß Beispiel Nr. | Ausgangswert [%] | Differenz nach 1 Woche [%] | Differenz nach 4 Wochen [%] | Differenz nach 12 Wochen [%] | Anmerkungen |
| 1 | 85.9 | | -0.4 | -2.3 | |
| 2 | 100 (titr.)¹⁾ | | ±0.0 .(titr.)¹⁾ | | Referenz- |
| | | | | | standard |
| | | | | | für HPLC |
| 3 | 87.2 | +1.1 | -1.1 | -5.0 | |
| 4 | 82.7 | -0.9 | -2.8 | -6.0 | |
| 5 | 93.7 | -0.5 | ±0.0 | -3.2 | |
| 6 | 83.1 | -4.8 | | | |
| 7 | 90.4 | -3.1 | | | |
| 8 | 85.3 | +1.2 | -6.0 | | |
| 9 | 86.1 | +1.8 | -4.1 | -9.5 | |
| 10 | 78.7 | -3.3 | -8.8 | | |
| 11 | 88.8 | -0.9 | -3.0 | -11.5 | |
| 12 | 75.0 | +0.3 | -5.3 | -5.6 | |
| 13 | 76.4 | +0.9 | -1.6 | -2.8 | |
| 14 | 92.9 | -0.5 | -4.5 | -10.4 | |
| 15 | 90.8 | -2.0 | -2.0 | -9.9 | |
| 16 | 94.6 | +1.4 | +0.1 | ±0.0 | |
| 17 | 90.5 | -0.8 | -5.6 | -12.6 | |
| 18 | 90.9 | +1.5 | +2.1 | -2.6 | |
| 21 | 98.4 | | -0.9 | -1.5** | ** 8 Wochen |
| 25 | 93.2 | | -3.7 | | |
| 26 | 97.7 | +0.2 | -2.7 | -5.1 | |
| 27 | 102.9 | +0.5 | -0.8 | -0.2 | |
| 28 | 95.9 | -0.7 | -1.1 | -4.7 | |
| 29 | 98.7 | +0.9 | +1.3 | +1.0 | |
| 30 | 98.6 | | -0.4 | | |
| freies | 91.5 | -17.9 | -24.25 | -28.4 | Vergleich |
| Hyperforin | | | | | |

| | | | | | |
|---|---|---|---|---|---|
| 1) Dieses Salz wird als Referenzstandard verwendet. Der Gehalt an Hyperforin (in % des berechneten stöchiometr. Werts) wurde mittels Perchlorsäure-Titration bestimmt. Die gefundenen Werte belegen die erhöhte Stabilität und verbes-serte Lagerfähigkeit der Hyperforin-Salze im Vergleich zum freien Hyperforin. | | | | | |

### Beispiel Nr. 33: Aktivierung der Proteinkinase PKCγ durch Hyperforin und dessen Salze.

Aktivatoren der PKC-γ sind potentiell geeignet, die α-Sekretase zu aktivieren. Solche Aktivatoren werden in Enzymtests gefunden, bei denen rekombinant produzierte PKC-γ suboptimal aktiv ist. Die Testmedien waren:

| | |
|---|---|
| HEPES-NaOH | 50 mM |
| EDTA | 1 mM |
| EGTA | 1,25 mM |
| MgCl₂ | 5 mM |
| DTT | 1mM |
| ATP | 0,1 µM |
| Histon III-S | 100 µg/ml |
| rekombinante PKC-γ | 200-100 ng/well |
| CaCl₂ | 1,32 mM |

In der nachfolgenden Tabelle Nr. III sind die Aktivitätssteigerungen der rekombinanten PKC-γ durch Hyperforin und dessen Salze in verschiedenen Konzentrationen aufgeführt.

**Tabelle Nr. III:**

| Aktivierung der Proteinkinase PKC-γ | | | |
|---|---|---|---|
| Aktivitätssteigerung der PKC-γ [%] bei der Substanz-Dosis: | | | |
| Beispiel Nr. | 10 µg/ml | 3 µg/ml | 1 µg/ml |
| Hyperforin | 45 | 51 | 6 |
| 1 | 56 | 30 | 9 |
| 5 | 48 | 20 | 3 |
| 21 | 56 | 22 | -9 |
| 24 | 21 | 24 | 7 |
| 25 | 45 | | 12 |
| 26 | 43 | 20 | 10 |
| 27 | 34 | 18 | 6 |
| 31 | 24 | 7 | 5 |
| 1 % DMSO | 6 | 5 | 4 |

In der Tabelle wurde die Kontrolle mit 100 nM Phorbol-12-myristat-13-acetat (TPA; Stimulator der PKC-γ) zu 100 % gesetzt.

Die ermittelten Werte zeigen einen deutlichen Anstieg der PKC-γ-Aktivität bei Zusatz von Hyperforin oder dessen Salzen zum Testmedium.

### Beispiel Nr. 34: Aktivierung der α1-Sekretase durch Hyperforin und dessen Salze.

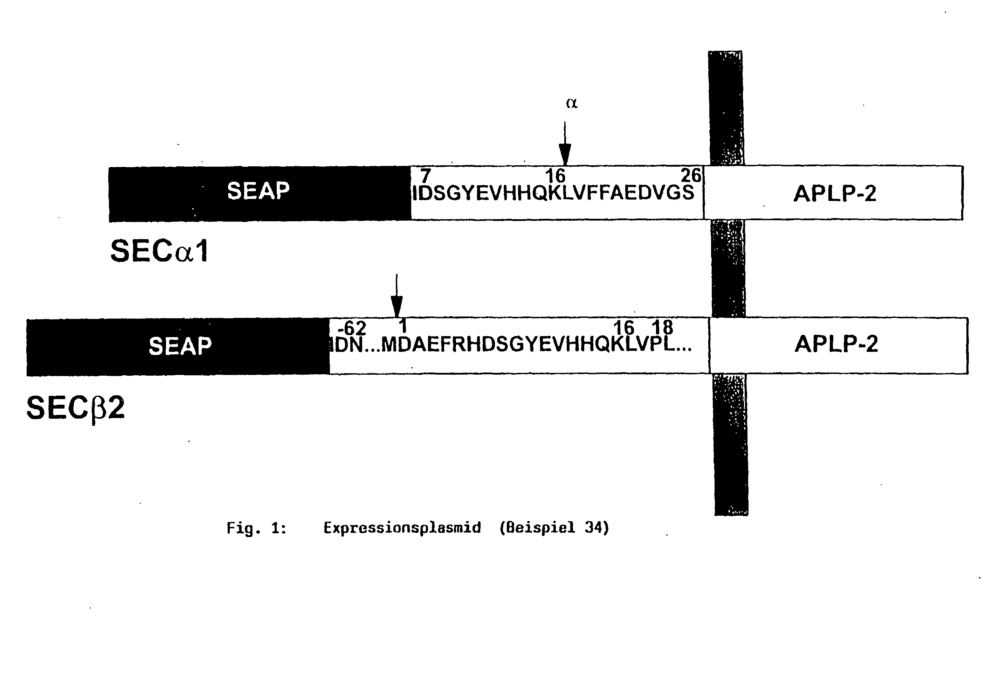

Um die α-Sekretaseaktivität in einem zellulären Testsystem unabhängig von der β- und γ-Sekretase nachweisen zu können, wurde ein Expressionsplasmid konstruiert, das aus DNA-Kassetten des APP (Alzheimer Precursor Protein), des APLP-2 (Alzheimer Precursor like Protein-2) sowie dem Reporterprotein Seap (Secreted alkaline phosphatase) zusammengesetzt ist (siehe Figur 1). Da die APP Kassette in beiden Konstrukten mit Aminosäure 7 der β-Amyloid Sequenz beginnt, fehlt die Erkennungssequenz für die β-Sekretase in den Sec-Fusionsproteinen. Die Spaltung durch die γ-Sekretase ist für das humane APLP-2 nicht beschrieben, so daß die Spezifität der Fusionsproteine für lediglich die α-Sekretase durch Austausch der Transmembrandomäne des APPs durch die des APLP-2, erreicht wird. Das Expressionsplasmid Secα1 wurde stabil in humane, neuronale Zellen SY5Y transfiziert. Aktivierung der α-Sekretase in diesen Secα1 transfizierten Zellen zeigt sich in einer vermehrten Abgabe der Seap in das Zellmedium. Die Seap Menge wird bestimmt und dient als Maß für die α-Sekretaseaktivität.

### Testbedingungen: 80 000 FL-2a Zellen/well. V = 100 µl. Inkubationszeit: 60 min. Konzentration der Substanzen: 10 µg/ml. Mittelwerte +/- S.D. von Triplikaten.

In der nachfolgenden Tabelle Nr. IV sind die in relativen Light-Units (RLU) gemessenen Mengen der Secreted alkaline phosphatase (Seap) als Maß für die Stimulierung der α-Sekretase-Aktivität durch Hyperforin und dessen Salze aufgeführt

**Tabelle Nr. IV:**

| Aktivierung der α-Sekretase | | | |
|---|---|---|---|
| Beispiel Nr. | Aktivität der α-Secretase [RLU] | S.D. [RLU] | Anmerkungen |
| Hyperforin | 441 | 55.1 | |
| 21 | 350 | 14.6 | |
| 24 | 445 | 67.1 | |
| 25 | 322 | 128.6 | |
| 26 | 423 | 34.4 | |
| 27 | 453 | 15.1 | |
| 28 | 457 | 42.9 | |
| 29 | 404 | 8.9 | |
| 30 | 419 | 41.9 | |
| 31 | 481 | 42.6 | |
| DMSO | 76 | 8.9 | Lösungsmittel-Kontrolle |
| TPA | 386 | 11.4 | Positiv-Kontrolle |

Als Positiv-Kontrolle wurde die Aktivierung der α-Sekretase durch den Phorbolester TPA (100 ng/ml) ermittelt.

Die ermittelten Werte zeigen eine starke Aktivierung der α-Secretase sowohl bei Zusatz von Hyperforin als auch dessen Salzen zum Testmedium.

## Patentansprüche

1. Salze des Hyperforins und Adhyperforins der allgemeinen Formel I
[A⁻]ₘ [B]^{p+} (I)
worin
m eine ganze Zahl von 1 bis 3,
[**A**⁻] ein Anion der Formel II mit n = 0 oder 1 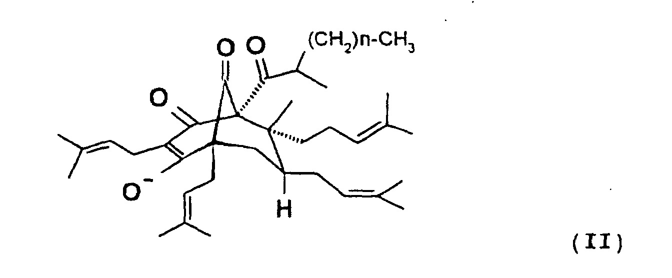 und [**B**]^{p+} ein Alkalimetallion oder ein Ammoniumion einer salzbildenden Stickstoffbase der allgemeinen Formel III ist, 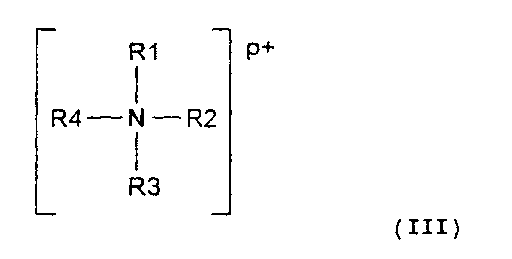 worin **R1, R2 und R3**
unabhängig voneinander ein Wasserstoffatom, eine geradkettige oder verzweigte Alkyl-, Cycloalkyl-, Bicycloalkyl-, Tricycloalkyl-, Alkenyl-, Alkinyl-, Heterocyclalkyl-, Aryl-, Heteroaryl, Arylalkyl- oder Heteroarylalkyl-Gruppe oder ein durch einen oder mehrere Hydroxy-, Alkoxy-, Aryloxy-, Alkanoyl-, Aroyl-, Carboxy-, Alkoxycarbonyl-, Amino-, Alkylamino-, Hydroxylamino-, Amido-, Carbamoyl-, Ureido-, Amidino-, Guanidino-, Cyano-, Azido-, Mercapto-, Alkylthio-, Alkylsulfoxy-, Alkylsulfonyl-, Alkylsulfenyl-, Aminosulfonyl-,
Fluor-, Chlor-, Brom-, Jod-, Alkyl- oder Perfluoralkyl-Rest(e) substituiertes Derivat der genannten Gruppen bedeuten,
oder worin **R1 und R2**
zusammen mit dem N-Atom einen Azetidin-, Pyrrolidin-, Pyrrolin-, Piperidin-, Piperazin-, Homopiperazin-, Morpholin-, Thiomorpholin-, Pyridin-, Di- oder Tetra-hydropyridin-, Pyrimidin-, Pyrazin-, Azepin-, Dihydroazepin-, Oxazepin-, Diazepin-, Imidazol-, Pyrazol-, Oxazol- oder Thiazol-Ring oder einen der genannten Ringe bedeuten, der ankondensierte aliphatische, heteroaliphatische, aromatische oder hetero-aromatische Ringe aufweist und/oder durch einen oder mehrere Hydroxy-, Alkoxy-, Aryloxy-, Alkanoyl-, Aroyl-, Carboxy-, Alkoxycarbonyl-, Amino-, Alkylamino-, Hydroxylamino-, Amido-, Carbamoyl-, Ureido-, Amidino-, Guanidino-, Cyano-, Azido-, Mercapto-, Alkylthio-, Alkylsulfoxy-, Alkylsulfonyl-, Alkylsulfenyl-, Aminosulfonyl-, Fluor-, Chlor-, Brom-, Jod-, Alkyl- oder Perfluoralkyl-Rest(e) substituiert ist,
und worin der Rest **R4**
ein Wasserstoffatom oder eine geradkettige oder verzweigte Alkylgruppe bedeutet,
wobei **p** = **m** ist und die Gesamtanzahl positiver Ladungen des Rests [B] angibt.

2. Salze nach Anspruch 1, worin das Alkalimetallion ein Lithium-, Natrium- oder Kaliumion ist.

3. Salze nach Anspruch 1, worin die salzbildende Stickstoffbase ausgewählt ist aus der Gruppe, bestehend aus aliphatischen und cycloaliphatischen Aminen, durch eine oder mehrere Hydroxylgruppen substituierten aliphatischen und cycloaliphatischen Aminen, Polyaminen, cyclischen und heterocyclischen Aminen, durch eine oder mehrere Niederalkyl- oder Hydroxyl-Reste substituierten cyclischen oder heterocyclischen Aminen, unsubstituierten und substituierten aromatischen, heteroaromatischen, arylaliphatischen und heteroarylaliphatischen Aminen, Aminosäureestern und Aminosäureamiden.

4. Salze nach Anspruch 3, worin die salzbildende Stickstoffbase N,N-Dicyclohexylamin ist.

5. Salze nach Anspruch 1 oder 3, worin die salzbildende Stickstoffbase ausgewählt ist aus der Gruppe, bestehend aus Wirkstoffen zur Therapie der Alzheimerschen Krankheit (AD), Wirkstoffen zur Therapie von Depressionen (Antidepressiva), Wirkstoffen zur Therapie von Angst- und Spannungszuständen (Anxiolytika), Calcium-antagonistisch wirkenden Stoffen mit basischer Seitenkette, Wirkstoffen zur Therapie von Dyspepsie, Prokinetika, β-Rezeptorenblocker und Nootropika.

6. Verfahren zur Herstellung der Salze des Hyperforins und Adhyperforins gemäss einem der Ansprüche 1 bis 5, bei dem Hyperforin und/oder Adhyperforin unter Inertgas in einem Lösungsmittel oder Lösungsmittelgemisch, ausgewählt aus der aus C5- bis C8-Alkanen oder Cycloalkanen, niederen Chloralkanen, Alkoholen, Ketonen, Estern und Ethern bestehenden Gruppe, gelöst werden und mit der Lösung einer Alkalimetallbase oder einer salzbildenden Stickstoffbase in einem der vorgenannten Lösungsmittel oder in Wasser im gewünschten stöchiometrischen Verhältnis vereint werden, wonach man das gebildete Salz auskristallisieren läßt und abtrennt oder die vereinten Lösungen evaporiert und nach Zugabe von Wasser lyophilisiert.

7. Verfahren zur Anreicherung bzw. Reingewinnung von Hyperforin und Adhyperforin in Form der Salze gemäss einem der Ansprüche 1 bis 5 aus Johanniskraut-Extrakten, **dadurch gekennzeichnet, dass** man einen Johanniskraut-Extrakt mit einem Gesamtgehalt an Hyperforin und Adhyperforin von 20-80 %, in einem geeigneten Lösungsmittel, das aus der aus apolaren C1-C10-Alkanen und -Cycloalkanen bestehenden Gruppe ausgewählt wird, löst und diese Lösung mit einer mindestens equimolaren Menge einer Alkalimetallbase oder einer salzbildenden Stickstoffbase oder einer Lösung einer solchen Base in einem der vorgenannten Lösungsmittel oder in einem C1-C4-Halogenalkan, Ether, Tetrahydrofuran oder Keton versetzt und das entstandene Salz abtrennt, reinigt und trocknet.

8. Verfahren nach Anspruch 7, **dadurch gekennzeichnet, dass** man als Base N,N-Dicyclohexylamin verwendet.

9. Verwendung von Salzen gemäß einem der Ansprüche 1 bis 5 zur stabilen Lagerhaltung von Hyperforin, Adhyperforin und deren Gemischen.

10. Pharmazeutische Zubereitung, enthaltend mindestens ein Salz gemäss einem der Ansprüche 1 bis 5.

11. Verwendung von Adhyperforin oder Gemischen aus Hyperforin und Adhyperforin zur Herstellung von pharmazeutischen Zubereitungen bzw. Arzneimitteln zur Behandlung der Alzheimerschen Krankheit und der damit einhergehenden Symptome.

## Claims

1. Salts of hyperforin and adhyperforin of general formula I
[**A**⁻]ₘ [**B**]^{P+} (I)
in which
m is an integer of from 1 to 3,
[**A**⁻] is an anion of formula II with n = 0 or 1 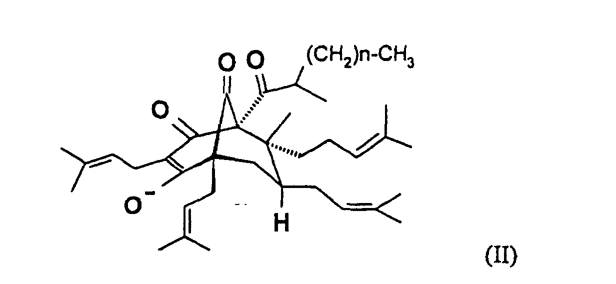
and [B]^{p+} is an ion of an alkali metal or an ammonium ion of a salt-forming nitrogen base of general formula **III** 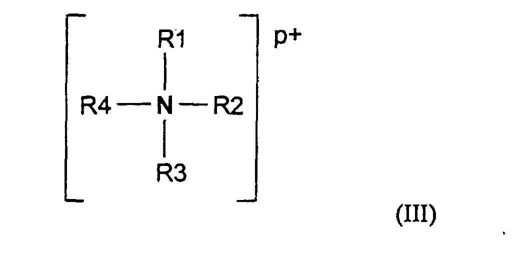
wherein **R1, R2 and R3**
independently of one another, are a hydrogen atom, a straight-chain or branched alkyl, cycloalkyl, bicycloalkyl, tricycloalkyl, alkenyl, alkynyl, heterocycloalkyl, aryl, heteroaryl, arylalkyl or heteroarylalkyl group, or a derivative of the said groups substituted with one or more hydroxy, alkoxy, aryloxy, alkanoyl, aroyl, carboxy, alkoxycarbonyl, amino, alkylamino, hydroxylamino, amido, carbamoyl, ureido, amidino, guanidino, cyano, azido, mercapto, alkylthio, alkylsulphoxy, alkylsulphonyl, alkylsulphenyl, aminosulphonyl, fluoro, chloro, bromo, iodo, alkyl or perfluoroalkyl residue(s),
or wherein the residues **R1 and R2**
together with the N atom are an azetidine, pyrrolidine, pyrroline, piperidine, piperazine, homopiperazine, morpholine, thiomorpholine, pyridine, di- or tetrahydropyridine, pyrimidine, pyrazine, azepine, dihydroazepine, oxazepine, diazepine, imidazole, pyrazole, oxazole, or thiazole ring, or one of the said rings which exhibits aliphatic, heteroaliphatic, aromatic or heteroaromatic rings condensed on to it and/or is substituted with one or more hydroxy, alkoxy, aryloxy, alkanoyl, aroyl, carboxy, alkoxycarbonyl, amino, alkylamino, hydroxylamino, amido, carbamoyl, ureido, amidino, guanidino, cyano, azido, mercapto, alkylthio, alkylsulphoxy, alkylsulphonyl, alkylsulphenyl, aminosulphonyl, fluoro, chloro, bromo, iodo, alkyl or perfluoroalkyl residue(s),
and wherein the residue **R4**
is a hydrogen atom or a straight-chain or branched alkyl group,
in which **p** = **m** and gives the total number of positive charges of the residue
[**B**].

2. Salts according to Claim 1 in which the alkali-metal ion is a lithium, sodium or potassium ion.

3. Salts according to Claim 1, in which the salt-forming nitrogen base is selected from the group consisting of aliphatic and cycloaliphatic amines, aliphatic and cycloaliphatic amines substituted with one or more hydroxyl groups, polyamines, cyclic and heterocyclic amines, cyclic or heterocyclic amines substituted with one or more lower alkyl or hydroxyl residues, unsubstituted and substituted aromatic, heteroaromatic, arylaliphatic and heteroarylaliphatic amines, amino acid esters and amino acid amides.

4. Salts according to Claim 3 in which the salt-forming nitrogen base is N,N-dicyclohexylamine.

5. Salts according to Claim 1 or 3 in which the salt-forming nitrogen base is selected from the group consisting of active substances for the therapy of Alzheimer's disease (AD), active substances for the therapy of depressive illnesses (antidepressants), active substances for the therapy of anxiety states and tension states (anxiolytics), substances acting as calcium antagonists with basic side chain, active substances for the therapy of dyspepsia, prokinetics, β-receptor blockers, and nootropics.

6. A method of manufacturing the salts of hyperforin and adhyperforin according to one of Claims 1 to 5, in which hyperforin and/or adhyperforin are dissolved under inert gas in a solvent or solvent mixture selected from the group consisting of C₅-C₈ alkanes or cycloalkanes, lower chloroalkanes, alcohols, ketones, esters and ethers, and combined in the desired stoichiometric ratio with the solution of an alkali-metal base or a salt-forming nitrogen base in one of the aforementioned solvents or in water, after which the salt formed is allowed to crystallise out and separated or the combined solvents are evaporated and lyophilised after addition of water.

7. A method of enriching or purely recovering hyperforin and adhyperforin in the form of the salts according to one of Claims 1 to 5 from St. John's wort extracts, **characterised in that** a St. John's wort extract with a total content of hyperforin and adhyperforin of 20-80%, is dissolved in a suitable solvent selected from the group consisting of apolar C₁-C₁₀ alkanes and C₁-C₁₀ cycloalkanes, and this solution is reacted with an at least equimolar quantity of an alkali-metal base or a salt-forming nitrogen base or a solution of one of these bases in one of the above-mentioned solvents or in a C₁-C₄ halogenoalkane, ether, tetrahydrofuran or ketone and the salt produced is separated, purified and dried.

8. A method according to Claim 7, **characterised in that** N,N-dicyclohexylamine is used as the base.

9. The use of salts according to one of Claims 1 to 5 for maintaining stable stocks of hyperforin, adhyperforin and their mixtures.

10. A pharmaceutical preparation containing at least one salt according to one of Claims 1 to 5.

11. The use of adhyperforin or mixtures of hyperforin and adhyperforin for manufacturing pharmaceutical preparations or medicinal products for the treatment of Alzheimer's disease and the symptoms associated with it.

## Revendications

1. Sels de l'hyperforine et de l'adhyperforine, de formule générale I
[A⁻]ₘ [B]^{p+} (I)
dans laquelle
m est un nombre entier compris entre 1 et 3,
[A⁻] est un anion de formule II où n = 0 ou 1 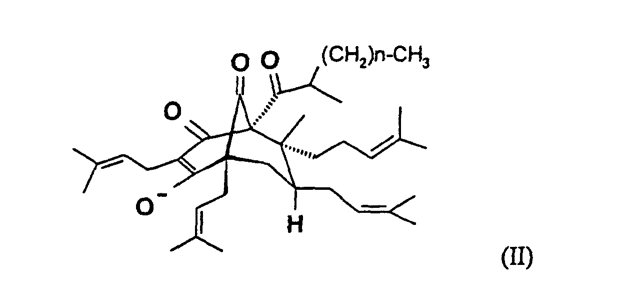
et [B]^{p+} est un ion de métal alcalin ou un ion ammonium d'une base azotée formant des sels, de formule générale III, 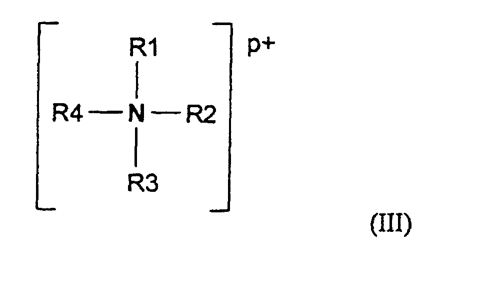
dans laquelle R1, R2, et R3 désignent indépendamment les uns des autres un atome d'hydrogène, un groupe alkyle, cycloalkyle, bicycloalkyle, tricycloalkyle, alcényle, alcinyle, hétérocyclalkyle, aryle, hétéroaryle, arylalkyle ou hétéroarylalkyle à chaîne droite ou ramifié ou un dérivé desdits groupes substitué par un ou plusieurs radicaux hydroxy, alcoxy, aryloxy, alcanoyle, aroyle, carboxy, alcoxycarbonyle, amino, alkylamino, hydroxylamino, amido, carbamoyle, uréido, amidino, guanidino, cyano, azido, mercapto, alkylthio, alkylsulfoxy, alkylsulfonyle, alkylsulfényle, aminosulfonyle, fluor, chlore, brome, iode, alkyle ou perfluoroalkyle ;
ou dans laquelle R1 et R2 désignent avec l'atome N un cycle azetidine, pyrrolidine, pyrroline, pipéridine, pipérazine, homopipérazine, morpholine, thiomorpholine, pyridine, dihydropyridine ou tétrahydropyridine, pyrimidine, pyrazine, azépine, dihydroazépine, oxazépine, diazépine, imidazol, pyrazol, oxazol ou thiazol ou desdits cycles, qui présente des cycles condensés aliphatiques, hétéroaliphatiques, aromatiques ou hétéro-aromatique et / ou qui est substitué par un ou plusieurs résidus hydroxy, alcoxy, aryloxy, alcanoyle, aroyle, carboxy, alcoxycarbonyle, amino, alkylamino, hydroxylamino, amido, carbamoyle, uréido, amidino, guanidino, cyano, azido, mercapto, alkylthio, alkylsulfoxy, alkylsulfonyle, alkylsulfényle, aminosulfonyle, fluor, chlore, brome, iode, alkyle ou perfluoroalkyle,
et dans laquelle le radical R4 désigne un atome d'hydrogène ou un groupe alkyle à chaîne droite ou ramifié ;
où p = m et indique le nombre global de charges positives du radical [B].

2. Sels selon la revendication 1, dans lesquels l'ion de métal alcalin est un ion lithium, sodium ou potassium.

3. Sels selon la revendication 1, dans lesquels la base azotée formant des sels est choisie dans le groupe se composant des amines aliphatiques et cycloaliphatiques, des amines aliphatiques et cycloaliphatiques substituées par un ou plusieurs groupes hydroxyles, des polyamines, des amines cycliques et hétérocycliques, des amines cycliques et hétérocycliques substituées par un ou plusieurs radicaux hydroxyle ou alkyle inférieur, des amines non substituées et substituées aromatiques, hétéroaromatiques, arylaliphatiques et hétéroarylaliphatiques, des esters d'acides aminés et des amides d'acides aminés.

4. Sels selon la revendication 3, dans lesquels la base azotée formant des sels est une amine N,N-dicyclohexyle.

5. Sels selon la revendication 1 ou 3, dans lesquels la base azotée formant des sels est choisie dans le groupe se composant de principes actifs destinés au traitement de la maladie d'Alzheimer (AD), de principes actifs destinés au traitement des dépressions (antidépresseurs), de principes actifs destinés au traitement des états de nervosité et d'angoisse (anxiolytiques), de substances ayant l'effet des antagonistes du calcium, à chaîne latérale basique, de principes actifs destinés au traitement de la dyspepsie, des prokinétiques, des bêta-bloquants et des nootropiques.

6. Procédé de fabrication des sels de l'hyperforine et de l'adhyperforine selon l'une des revendications 1 à 5, dans lequel l'hyperforine et / ou l'adhyperforine sont dissoutes sous un gaz inerte dans un solvant ou un mélange de solvants, choisi parmi le groupe se composant des alcanes ou cycloalcanes en C5 à C8, des chloroalcanes inférieurs, des alcools, des cétones, des esters et des éthers, et sont combinés avec la solution d'une base de métal alcalin ou d'une base azotée formant des sels dans l'un des solvants précédemment cités ou dans l'eau selon une proportion stoechiométrique désirée, après quoi on laisse le sel formé se cristalliser, et on le sépare, ou on évapore les solutions combinées et on les lyophilise après addition d'eau.

7. Procédé d'enrichissement, respectivement de préparation sous forme pure, d'hyperforine et d'adhyperforine sous forme de sels selon l'une des revendications 1 à 5, à partir d'extraits de millepertuis,
**caractérisé en ce que** l'on dissout un extrait de millepertuis présentant une teneur totale en hyperforine et en adhyperforine compris entre 20 et 80 %, dans un solvant approprié, qui est choisi dans le groupe se composant des alcanes apolaires en C1 - C10 et des cycloalcanes apolaires en C1-C10, et on ajoute à cette solution une quantité au moins équimolaire d'une base de métal alcalin ou d'une base azotée formant des sels saline ou d'une solution d'une telle base dans un des solvants sus-cités ou dans un halogénoalcane en C1-C4, un éther, un tétrahydrofurane ou une cétone et on sépare, purifie et sèche le sel produit.

8. Procédé selon la revendication 7, **caractérisé en ce qu'**on utilise comme base l'aminé N,N-dicyclohexyle.

9. Utilisation des sels selon l'une des revendications 1 à 5 pour un stockage stable de l'hyperforine et de l'adhyperforine et de leur mélanges.

10. Préparation pharmaceutique, comprenant au moins un sel selon l'une des revendications 1 à 5.

11. Utilisation de l'adhyperforine ou de mélanges de l'adhyperforine et de l'hyperforine pour la fabrication de préparations pharmaceutiques, respectivement de médicaments, pour le traitement de la maladie d'Alzheimer et des symptômes qu'elle génère.
